# FASCICULE DE BREVET EUROPEEN

(11) **EP 1 061 888 B1**
(45) Date de publication et mention de la délivrance du brevet: **24.11.2004**
(21) Numéro de dépôt: 99950817.9
(22) Date de dépôt: 22.10.1999
(51) Int. Cl.: A61K 6/02

(54) **BIOMATERIAU POUR LE TRAITEMENT DES PERIODONTOPATHIES**
BIOMATERIAL ZUR BEHANDLUNG VON ZAHNFÄULE
BIOLOGICAL MATERIAL FOR TREATING PERIODONTAL DISEASES

(30) Priorité: 23.10.1998 GB 9823307
(43) Date de publication de la demande: 27.12.2000
(73) Titulaire: SCS FRASSANITO et Cie, 98000 Monaco (MC)
(72) Inventeur: RIGOULET, Franck, F-06410 Biot (FR)
(74) Mandataire: Bonneau, Gérard
(86) Numéro de dépôt international: PCT/FR1999/002582
(87) Numéro de publication internationale: WO 2000/024363

(56) Documents cités:
- WO-A-94/17838
- WO-A-94/26283
- WO-A-97/46178
- FR-A- 2 637 502

## Description

### Domaine technique

La présente invention concerne un biomatériau pour le traitement des périodontopathies et toutes les affections associées, ce biomatériau comprenant de la bioaragonite biocompatible.

### Etat de la technique

Les périodontopathies ou maladies du périodonte, peuvent être d'origine diverse, comme par exemple génétique, infectieuse ou accidentelle.

Au sens de la présente invention, on entend par "bioaragonite" la nacre, de préférence sous forme micronisée.

La nacre ou aragonite chonchylifère, est en effet une formation minéralisée biogène, constituée essentiellement d'une partie minérale consistant en du carbonate de calcium cristallisé exclusivement sous forme d'aragonite et essentiellement d'une matrice organique composée de substances fibreuses et non fibreuses, représentant environ 1 à 2% de la masse totale. La nacre peut être obtenue à partir de la coquille de mollusques bivalves, notamment de certaines huîtres perlières telles que *Pinctada maxima,* dont elle constitue la couche la plus interne.

Le document WO 90/14111, par exemple, divulgue des implants dentaires à base de la couche interne de coquilles de mollusques aquatiques.

De même, la publication "La nacre au service du squelette humain" (E.P. LOPEZ, S..BERLAND et A. LE FAOU, La Recherche 262, Février 1994, Vol. 25, pages 208 à 210) décrit un implant à base de nacre qui, placé dans l'os de la mâchoire, est bio-intégré. Il en résulte, entre l'os et la racine dentaire artificielle, une relation physiologique qui fait que la dent est ressentie comme une dent naturelle.

A également été décrite une intervention permettant de combler la perte osseuse et consistant à mélanger de la poudre de nacre à une goutte de sang du malade, pour former une pâte avec laquelle on colmate la lacune osseuse. Une telle intervention peut être utilisée par exemple en stomatologie ("La nacre au secours des os ; nacre et stomatologie, l'alternative au dentier", Sciences et Avenir, Octobre 1994, p. 42).

Cependant, une telle intervention ne donne pas de résultats satisfaisants car le matériau de comblement obtenu (avec la poudre de nacre mélangée au sang) se délite dès qu'il est placé dans le site opératoire. Par ailleurs, la nacre utilisée pure peut entraîner une stimulation excessive de la formation des os, résultat évalué scientifiquement chez l'animal.

### Exposé de l'invention

Le but de l'invention est donc de fournir un biomatériau pour le traitement des périodontopathies et affections associées qui ne présente pas les inconvénients cités ci-dessus.

Le biomatériau selon l'invention, comprend de la bioaragonite biocompatible, du carbonate de calcium ajouté et éventuellement un liant.

### Description de l'invention

La bioaragonite biocompatible est présente dans une proportion de 62 à 98%, préférentiellement dans une proportion de 70 à 90% et plus préférentiellement encore de 75 à 85%, par rapport au poids total du biomatériau.

La bioaragonite biocompatible est obtenue par inhibition de la partie la plus immunogène de la substance organique de la bioaragonite, afin de ne maintenir bioactive que la partie de la substance organique en association avec le minéral susceptible d'avoir un effet positif.

Mais la bioaragonite biocompatible ainsi obtenue ne peut pas être utilisée, en tant que telle, pour le traitement des périodontopathies et affections associées.

La Société Demanderesse a découvert, de façon surprenante, qu'il convenait en effet de rajouter du carbonate de calcium à cette bioaragonite biocompatible, afin de rendre celle-ci facilement utilisable en clinique.

La Société Demanderesse mélange donc cette bioaragonite biocompatible avec du carbonate de calcium pur sous forme pulvérulente. Ce carbonate de calcium peut se présenter sous forme cristalline, sous la forme de calcite (carbonate de calcium orthorhombique), soit sous la forme d'aragonite (carbonate de calcium rhomboédrique) ou bien sous forme amorphe. Cet ajout de carbonate de calcium permet de rendre la pâte de bioaragonite biocompatible, modelable avec le sang postérieurement ajouté. En outre, le fait de mélanger la bioaragonite biocompatible (constituée essentiellement de carbonate de calcium pur cristallin sous forme orthorhombique) avec du carbonate de calcium pur, permet d'éviter une repousse osseuse excessive. L'utilisation de la bioaragonite biocompatible seule entraînerait en effet un tel inconvénient. Le carbonate de calcium ajouté est présent dans une proportion de 2 à 38% en poids, préférentiellement de 10 à 30% et plus préférentiellement encore de 15 à 25% par rapport au poids total du biomatériau. Le mélange entre la bioaragonite biocompatible et le carbonate de calcium ajouté s'effectue dans un mélangeur puis ce mélange est autoclavé à une température de 100 à 120°C pendant 15 à 20 min. Le stockage de l'ensemble s'effectue ensuite dans des sacs stériles ou dans des flacons stérilisés, afin d'éviter toute contamination exogène.

La mise en oeuvre de l'invention (c'est à dire l'administration du biomatériau au patient) nécessite que soit ajouté à cet ensemble bioaragonite biocompatible - carbonate de calcium ajouté, un certain nombre d'adjuvants.

A cet ensemble bioaragonite biocompatible - carbonate de calcium ajouté, peut être additionné un liant qui est présent dans une proportion de 6% à 15% en poids par rapport au poids total du biomatériau, préférentiellement dans une proportion de 7% à 12% et plus préférentiellement encore de 8% à 10%. Ce liant est choisi préférentiellement parmi l'acide hyaluronique, l'acide chondroïtine sulfurique, la gomme de guar, un alginate, la gomme xanthane ou le collagène, et particulièrement le collagène marin. Ce liant évite à l'ensemble bioaragonite biocompatible + carbonate de calcium ajouté de se déliter au contact du site opératoire hyperhémié.

Est également ajouté à cet ensemble un plastifiant, constitué par une substance biocompatible, susceptible de produire un ensemble homogène pénétrable par les liquides biologiques circulants. Ce plastifiant est ajouté dans des quantités variables suivant le choix du praticien d'obtenir une pâte plus ou moins dure. Préférentiellement, le plastifiant consiste en du sang autologue. Ce sang est prélevé à l'aide d'une seringue stérile sur le site opératoire, dans les lacunes de l'os spongieux constituant le parodonte ou préférentiellement encore en prélèvement intraveineux. Ce plastifiant permet de produire une pâte homogène adhérente plastique ne se délitant pas dans le site où la pâte est destinée à être introduite, et pénétrable par les liquides circulants.

Un durcisseur peut également être ajouté. Ce durcisseur est ajouté dans des quantités variables suivant le choix du praticien d'obtenir un durcissement plus ou moins rapide. Ce durcisseur permet de solidifier l'ensemble homogène introduit sur le lieu de l'affection comme définie. Dans le cas où le sang autologue est choisi en tant que plastifiant, celui-ci jouera également le rôle de durcisseur.

La dénaturation ménagée de la bioaragonite s'effectue avec comme matière première de départ la couche la plus interne de la coquille de mollusques bivalves, préférentiellement présentée sous forme de tablettes de 2 à 5 cm de longueur et de 0,3 cm d'épaisseur.

Cette matière première subit donc tout d'abord une opération de décontamination et d'oxydation et un lavage qui sont réalisés dans des récipients en matière inerte supportant la chaleur. La matière première est tout d'abord portée à ébullition dans de l'eau déminéralisée pendant environ une heure, puis on y ajoute le liquide décontaminant, préférentiellement dans des proportions de ¼ en poids (¼ de liquide décontaminant pour ¾ d'eau). Ce liquide décontaminant consiste préférentiellement en un mélange antiseptique et oxydant en solution aqueuse d'hypochlorite de sodium à 6,6 % de chlore actif. L'ébullition est maintenue pendant plusieurs heures, avec brassages. La matière première est ensuite rincée très soigneusement avec brassages sous courant d'eau jusqu'à élimination complète du liquide décontaminant et autres impuretés.

La matière première est ensuite rincée à l'eau déminéralisée et subit plusieurs ébullitions successives avec rinçages.

La matière première est ainsi rendue biocompatible, puisque la partie la plus immunogène de la substance organique a été inactivée au cours de la décontamination oxydative.

Lors d'une deuxième étape, la matière première biocompatible ainsi obtenue est séchée en atmosphère contrôlée à environ 100°C.

Dans une étape suivante, la matière première biocompatible sèche ainsi obtenue est stockée dans des sacs plastiques stériles. Puis elle est concassée dans des sacs et subit un meulage jusqu'à réduction sous forme pulvérulente. Le meulage se fait dans des récipients spécifiques réservés exclusivement à cet effet.

La matière première biocompatible pulvérulente (que l'on appellera ici bioaragonite biocompatible) ainsi obtenue est stockée dans des sacs en plastique, type sac poupinel, permettant la dernière phase de lavage.

La bioaragonite biocompatible est enfin lavée plusieurs fois dans les sacs à l'eau distillée. Puis elle est séchée au four Pasteur. Elle est ensuite réduite en poudre par concassage au maillet.

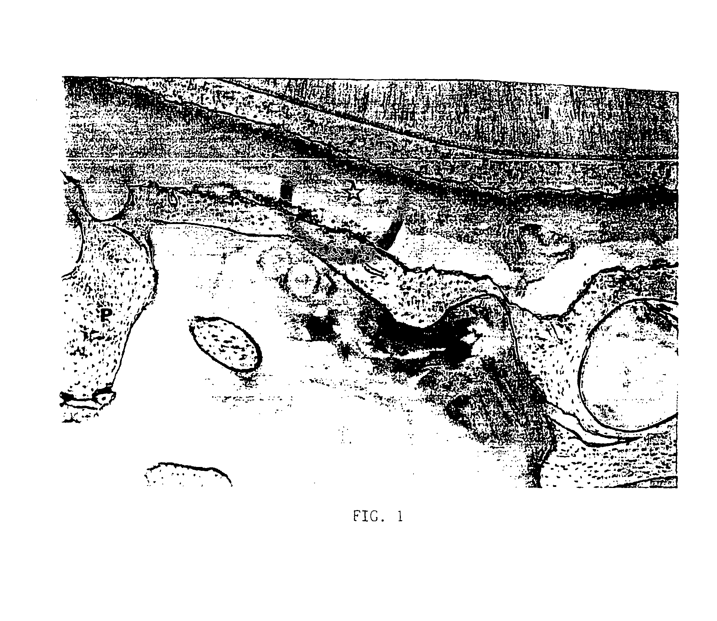

L'efficacité d'un tel biomatériau dans le traitement des périodontopathies et affections associées est démontrée par les résultats des expérimentations suivantes, et illustrée dans les figures 1 à 14 qui représentent des coupes histologiques de tissus, dans lesquelles (**P**) correspond à l'os parodontal, (**L**) correspond au ligament alvéolo-dentaire, (**I**) correspond à l'ivoire et (**N**) correspond aux particules de nacre.

Des prélèvements sont pratiqués chez le chien sur des dents présentant des lésions du périodonte provoquées expérimentalement, traitées avec le biomatériau selon l'invention et non traitées (servant de témoin).

On entend par périodonte, les tissus qui entourent et constituent le support de la dent : os parodontal, ligament alvéolo-dentaire et cément.

Les échantillons sont traités selon les techniques utilisées pour l'étude histologique des tissus minéralisés sans déminéralisation préalable.
Les figures 1 à 4 représentent des coupes histologiques de l'os parodontal et du ligament alvéolo-dentaire après induction d'une parodontopathie.
Les figures 5 et 6 représentent des coupes histologiques du ligament alvéolo-dentaire après induction d'une parodontopathie.
Les figures 7 et 8 représentent des coupes histologiques de l'os parodontal après induction d'une parodontopathie et traitement avec le biomatériau selon l'invention.
Les figures 9 et 10 représentent des coupes histologiques de l'os parodontal et du ligament alvéolo-dentaire après induction d'une parodontopathie et traitement avec le biomatériau selon l'invention.
La figures 11 représente une coupe histologique du ligament alvéolo-dentaire après induction d'une parodontopathie et traitement avec le biomatériau selon l'invention.
Les figures 12 et 13 représente des coupes histologiques du ligament alvéolo-dentaire plus particulièrement au niveau de l'interface os parodontal / ligament alvéolo-dentaire, après induction d'une parodontopathie et traitement avec le biomatériau selon l'invention.
La figures 14 représente une coupe histologique du cément plus particulièrement au niveau de l'interface ligament alvéolo-dentaire / dent, après induction d'une parodontopathie et traitement avec le biomatériau selon l'invention.

Les 3 tissus constituant le périodonte, à savoir l'os parodontal, le ligament alvéolo-dentaire et le cément, ont fait l'objet d'une étude approfondie en microscopie optique.

L'os parodontal constitue la paroi de l'alvéole, il est de type spongieux.

L'os parodontal d'une dent présentant une périodontopathie provoquée, est le siège d'une dégénérescence importante se traduisant par la présence de larges cryptes nécrotiques endoosseuses remplaçant les petites lacunes actives trouvées dans un os spongieux sain de ce type.

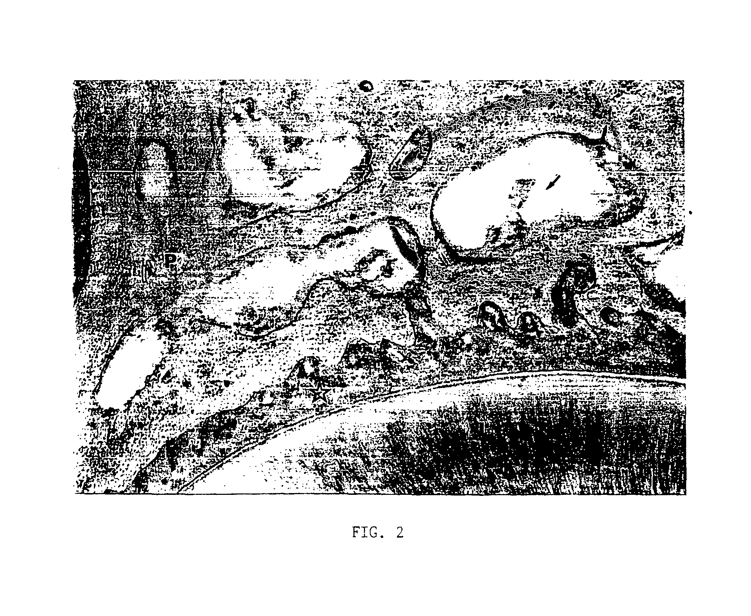

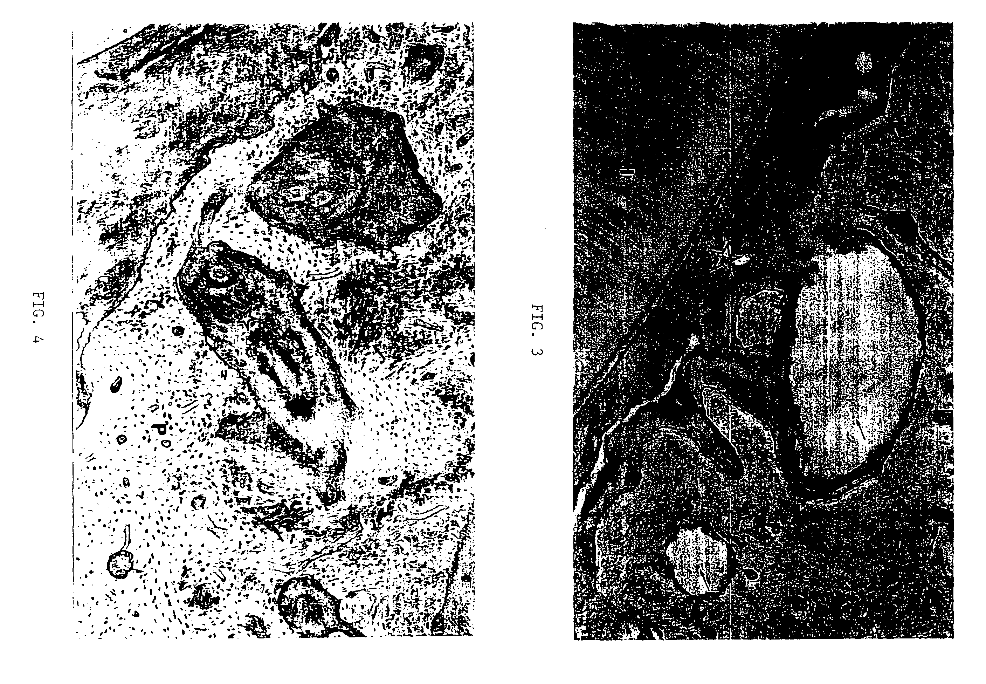

Les cryptes de résorption au contact du ligament alvéolo-dentaire sont nombreuses et anormalement agrandies. On observe dans cet os une absence d'unités actives de remodelage caractérisant un os physiologiquement sain. Il n'existe plus de relation physiologique fonctionnelle entre la paroi alvéolaire, le ligament alvéolo-dentaire et la surface de la racine pauvre ou dépourvue de cément. Les faisceaux de fibres de Sharpey attachant la dent à l'os parodontal ont disparu, et sont remplacées par de nombreux îlots fibreux désorganisés. Ces observations sont illustrées par les figures 1 à 4. A la figure 1, on observe une lésion ( 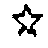 ) induite au niveau de l'os parodontal et une absence de ligament alvéolo-dentaire. A la figure 2, on observe un ligament alvéolo-dentaire désorganisé ( 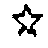 ) et un os parodontal anormalement vacuolaire. Les vacuoles sont caractérisées par une absence de population cellulaire (→). A la figure 3, on observe d'importantes cryptes de résorption (→) au niveau de l'os parodontal et un ligament désorganisé ( 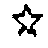 ). A la figure 4, sont représentées en détail les cryptes de résorption ( 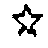 ) remplies de cellules pycnotiques au niveau de l'os parodontal. On observe également des îlots d'os retirés (→).

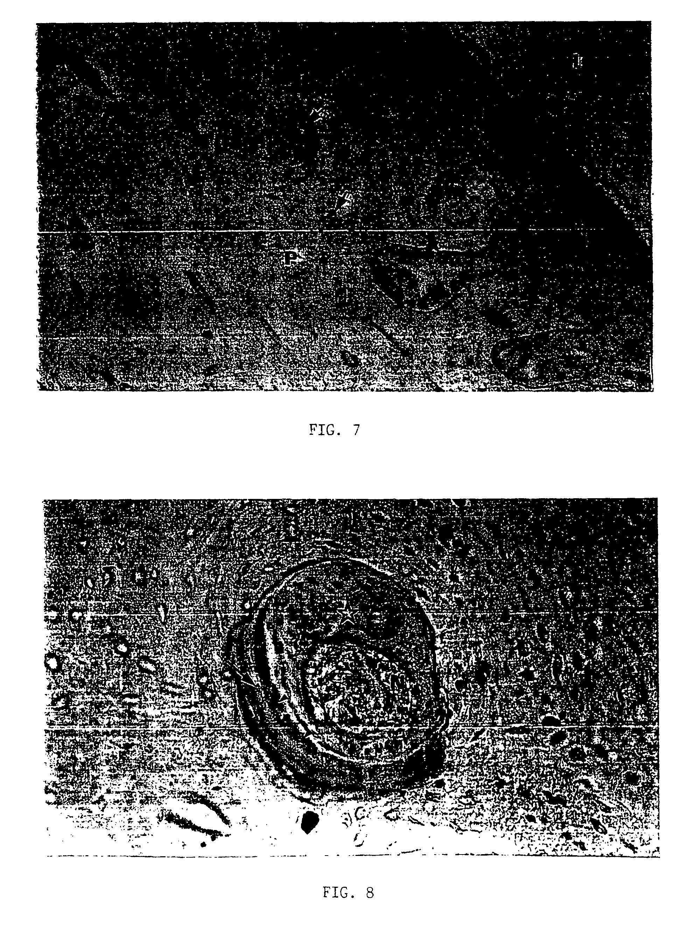

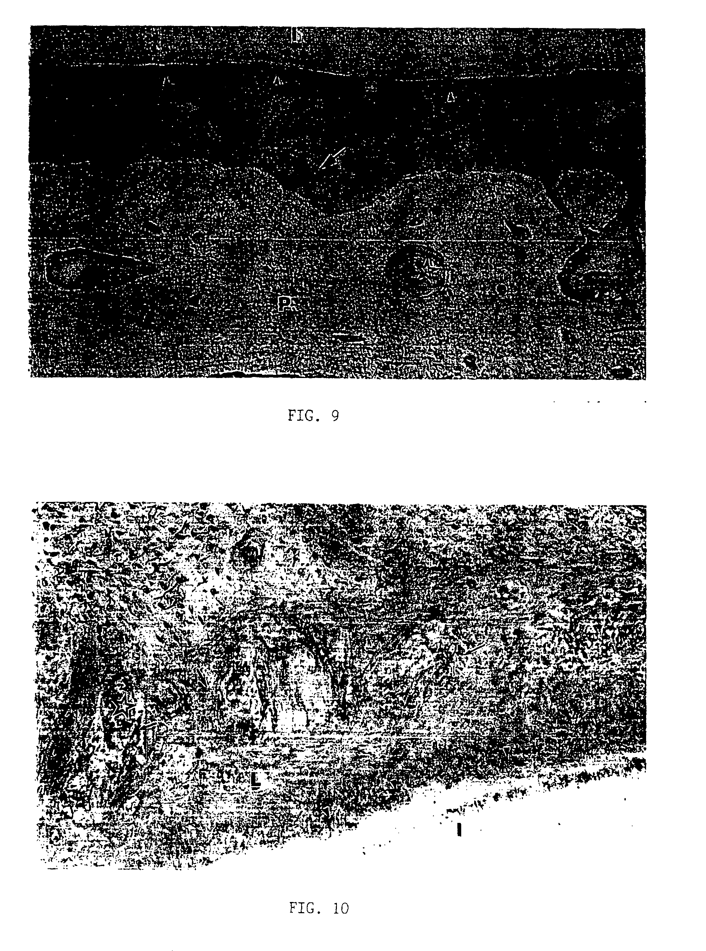

L'os parodontal d'une dent traitée par utilisation du biomatériau selon l'invention, ne présente plus de cryptes de dégénérescence. Celles-ci sont restructurées par apposition d'os nouveaux. L'os est régulièrement lamellaire témoignant de sa maturité physiologique et est le siège d'un remodelage actif. Les unités de remodelage sont bordées par des cellules formatrices d'os (ostéoblastes) très activées et par des bordures d'os en cours de minéralisation témoignant d'une néoformation osseuse. Une excellente cohésion cellulaire y est observée entre la paroi de l'os alvéolaire et la racine enrichie en cément. Les îlots fibreux désorganisés ont disparu et sont remplacés par des faisceaux de fibres de Sharpey restaurant l'attachement de la dent à l'os parodontal. Cette cohésion se traduit par un aspect histo-structural, démontrant l'intégrité du périodonte et notamment le rôle actif de l'un des ses éléments : le ligament alvéolo-dentaire. Les figures 7 à 10 illustrent bien ces observations. A la figure 7, l'os parodontal est le siège d'un remodelage actif (→). A la figure 8, on peut voir le détail d'une unité fonctionnelle de remodelage ( 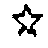 ) organisée autour d'un fragment de nacre subissant une biodissolution. On peut noter le bord ostéoïde significatif (→) subissant un procédé de minéralisation. On peut noter également les preuves de formation osseuse et la présence d'ostéoblastes actifs ( 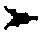 ). A la figure 9, l'os parodontal est le siège d'un actif remodelage ( 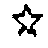 ). On note une organisation claire au niveau du ligament alvéolo-dentaire, des amas de fibres de Sharpey ancrées dans l'os parodontal et le cément (→), une bordure de cellules activées reconstruisant le cément ( 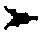 ). A la figure 10, on voit en détail la qualité physiologique de l'os parodontal et du ligament alvéolo-dentaire. On note la présence de fibroblastes activés, de fibres de Sharpey (→), en contact avec des particules de nacres subissant une biodissolution ( 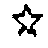 ), et une bordure de cémentoblastes actifs.

En ce qui concerne le ligament alvéolo-dentaire et le cément, un ligament alvéolo-dentaire sain est constitué par un tissu conjonctif fibreux dense reliant la racine de la dent à l'alvéole osseuse (os parodontal). L'ivoire de la racine est recouvert par une lame de cément synthétisée par une couche de cellules, les cémentoblastes, qui une fois pris dans le cément lorsque celui-ci est physiologiquement actif, se transforment en cémentocytes situés dans des lacunes. Le cément est peu diffèrent de l'os. Il joue un rôle primordial. En effet, les fibres de collagène du ligament alvéolo-dentaire sont groupées en faisceaux appelés fibres de sharpey. Ces faisceaux partent de la paroi du parodonte et viennent s'ancrer dans le cément. Le ligament alvéolo-dentaire sain est riche en vaisseaux sanguins et en filets nerveux. Des amas de cellules épithéliales soutiennent le trajet des fibres de Sharpey. Ils forment les corpuscules de Malassez.

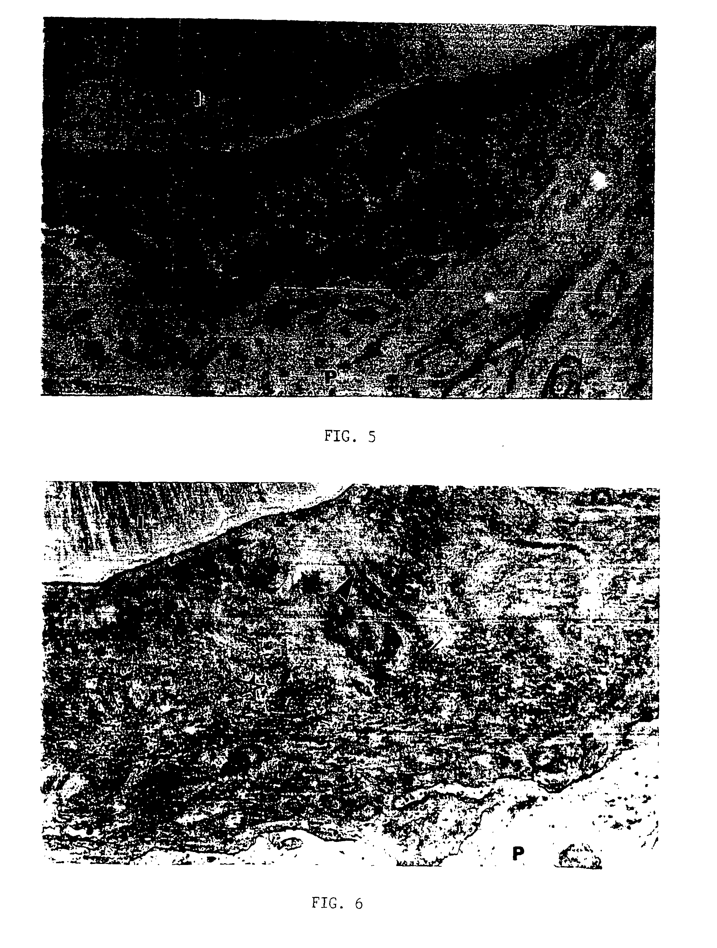

En ce qui concerne un ligament alvéolo-dentaire et un cément d'une dent présentant une affection du périodonte, on observe que le ligament alvéolo-dentaire en certains points est inexistant, c'est à dire totalement lysé. Lorsqu'il subsiste, il est constitué par un tissu hétérogène dont les composantes sont difficilement identifiables. Les fibroblastes sont en dégénérescence. L'irrigation sanguine est inexistante. Les faisceaux de fibres de Sharpey sont désorganisés et ne présentent pas de point d'attache, ni sur l'os parodontal, ni sur le cément pratiquement inexistant. On note une absence totale de cémentoblastes actifs. Les figures 5 et 6 illustrent bien ces observations. A la figure 5, on observe un ligament désorganisé ( 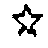 ) composé de tissu conjonctif hétérogène. Les fibres de Sharpey sont sans point d'attache (→). A la figure 6, on observe un détail du ligament alvéolo-dentaire ( 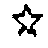 ), des fibroblastes pycnotiques (→), des fibres de Sharpey sans point d'attache ( 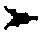 ), une fine couche de cément avec absence de cémentoblastes actifs ( 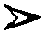 ).

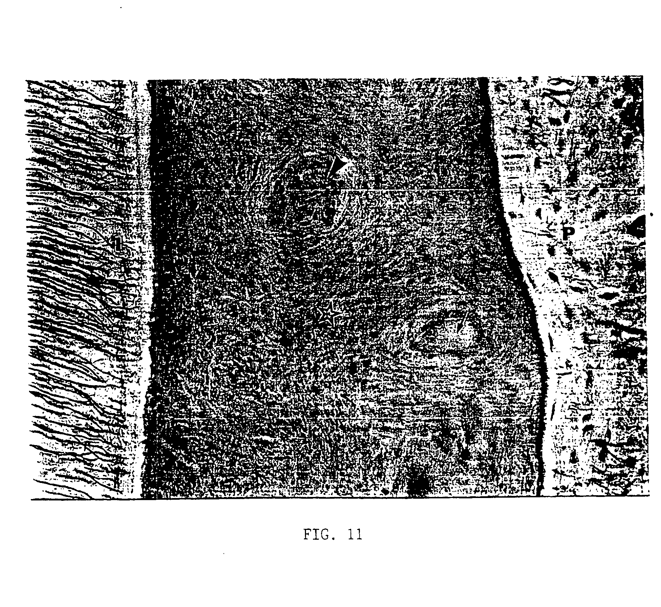

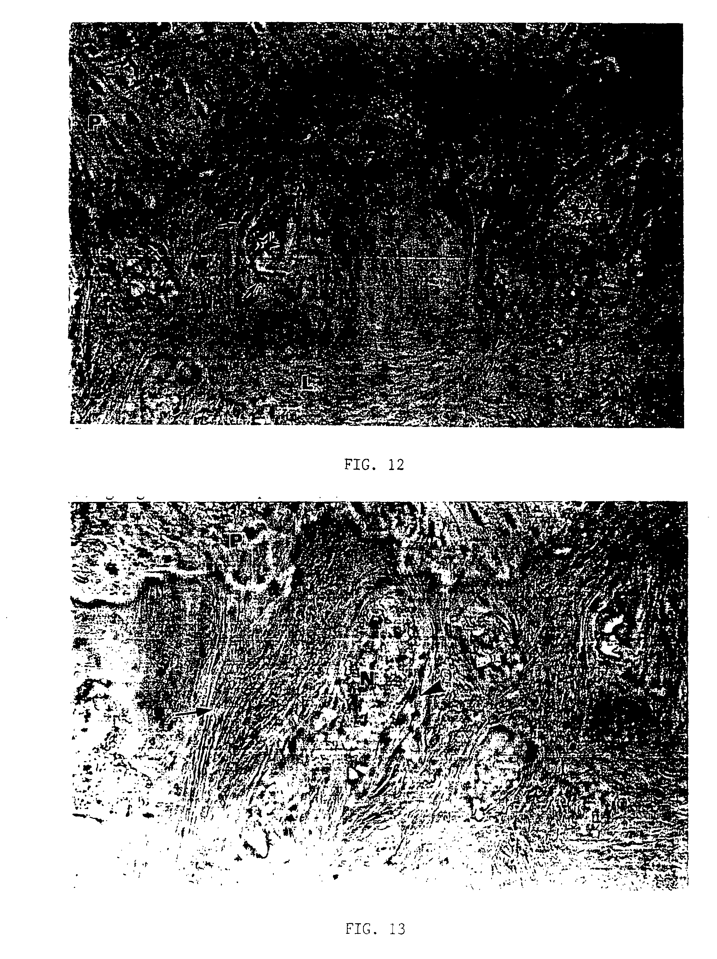

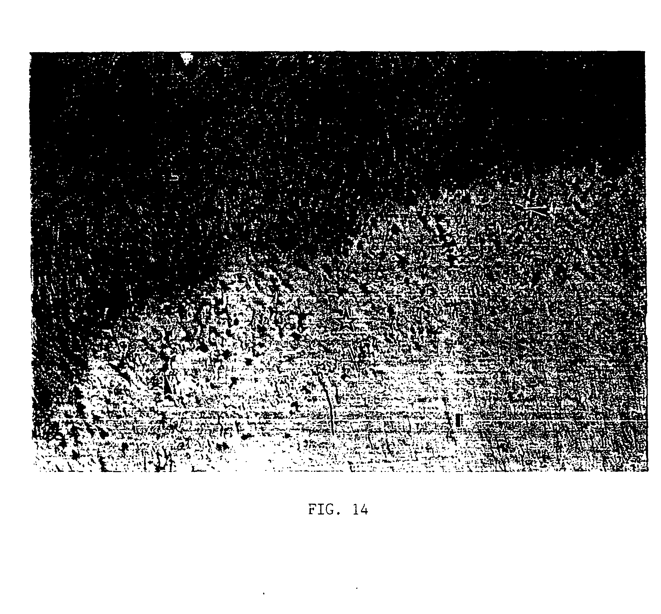

En ce qui concerne en revanche le ligament alvéolo-dentaire et le cément d'une dent traitée avec le biomatériau selon l'invention, tels que représentés aux figures 11 à 14, l'os parodontal régénéré riche en ostéocytes actifs, envoie de larges faisceaux de fibres de Sharpey qui viennent s'ancrer sur une couche de cément néoformée. On observe une zone de cémentoblastes actifs et un cément riche en cémentocytes en parfaite cohésion physiologique avec l'ivoire sous-jacent. Les fibroblastes sont très activés, comme en témoignent leurs noyaux clairs et renflés, tout particulièrement autour des particules du biomatériau d'implantation résiduel, en cours de bio-dissolution. Le ligament alvéolo-dentaire est riche en vaisseaux sanguins et en corpuscules de Malassez entourés de fibres de Sharpey. La figure 11 montre la qualité physiologique des tissus : capillaire sanguin (→). On observe les corpuscules de Malassez ( 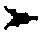 ) et les cémentoblastes actifs ( 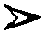 ). La figure 12 montre en détail l'activation des fibroblastes (→) en contact avec les particules de nacre subissant un processus de biodissolution ( 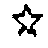 ). On observe également de nombreux amas de fibres de Sharpey ( 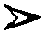 ). La figure 13 montre l'organisation des fibres de Sharpey (→), des amas de fibres de Sharpey ancrées dans l'os parodontal, près d'une particule de nacre subissant un processus de biodissolution et des fibroblastes activés ( 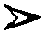 ). A la figure 14, on observe en détail la bordure de cémentoblastes actifs (→) et les cémentocytes ( 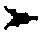 ) piégés sous la couche nouvellement formée ( 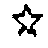 ).

Ces expérimentations permettent donc de conclure que le traitement des affections du périodonte avec le biomatériau selon l'invention, provoque une restructuration de l'os parodontal et une stimulation de son activité, une stimulation des cémentoblastes et cémentocytes, avec synthèse accrue de cément et une régénération fonctionnelle du ligament alvéolo-dentaire, notamment des fibres de Sharpey et de leur point d'attache os parodontal - cément.

Ces différents effets permettent la restauration complète de l'activité fonctionnelle de la dent au sein de son alvéole.

## Revendications

1. Biomatériau pour le traitement des périodontopathies et toutes les affections associées, **caractérisé par le fait qu'**il comprend :
- de la bioaragonite biocompatible,
- du carbonate de calcium pur ajouté sous forme cristalline ou sous forme amorphe,
- éventuellement un liant.

2. Biomatériau selon la revendication 1, **caractérisé par le fait que** le carbonate de calcium pur ajouté se présente sous forme pulvérulente.

3. Biomatériau selon l'une des revendications 1 ou 2, **caractérisé par le fait que** la bioaragonite biocompatible est présente dans une proportion de 62 à 98% en poids, préférentiellement dans une proportion de 70 à 90% et plus préférentiellement encore de 75 à 85% par rapport au poids total du biomatériau.

4. Biomatériau selon l'une des revendications 1 à 3, **caractérisé par le fait que** le carbonate de calcium pur ajouté est présent dans une proportion de 2 à 38% en poids, préférentiellement dans une proportion de 10 à 30% et plus préférentiellement encore de 15 à 25% par rapport au poids total du biomatériau.

5. Biomatériau selon l'une quelconque des revendications 1 à 4, **caractérisé par le fait que** le liant est choisi parmi l'acide hyaluronique, l'acide chondroïtine sulfurique, la gomme de guar, un alginate, la gomme xanthane ou le collagène.

6. Biomatériau selon l'une quelconque des revendications 1 à 5, **caractérisé par le fait que** le liant est présent dans une proportion de 6% à 15% en poids, préférentiellement dans une proportion de 7% à 12% et plus préférentiellement encore de 8% à 10% par rapport au poids total du biomatériau.

7. Biomatériau selon l'une quelconque des revendications 1 à 6, **caractérisé par le fait que** la bioaragonite biocompatible est constituée par la couche la plus interne de la coquille de mollusques bivalves sous forme micronisée et dans laquelle la partie immunogène de la substance organique a été inactivée.

8. Biomatériau selon l'une quelconque des revendications 1 à 7, **caractérisé par le fait que** la bioaragonite biocompatible est obtenue à l'aide d'une dénaturation ménagée de la couche interne de la coquille de mollusques bivalves, dénaturation telle que la partie la plus immunogène de la substance organique de cette couche interne soit inactivée et que la partie de la substance organique en association avec le minéral susceptible d'avoir un effet positif soit essentiellement préservée, suivie d'un rinçage, d'un séchage et d'un concassage jusqu'à obtention d'une forme pulvérulente.

9. Biomatériau selon la revendication 8, **caractérisé par le fait que** la dénaturation ménagée consiste en une oxydation de la partie la plus immunogène de la substance organique par de l'hypochlorite.

10. Utilisation d'un mélange bioaragonite biocompatible + carbonate de calcium pur ajouté sous forme cristalline ou sous forme amorphe pour la fabrication d'un biomatériau destiné au traitement des périodontopathies et toutes les affections associées.

11. Méthode de préparation d'une composition contenant de la bioaragonite biocompatible pour le traitement des périodontopathies et toutes les affections associées, qui consiste à mélanger de la bioaragonite biocompatible avec du carbonate de calcium pur sous forme cristalline ou sous forme amorphe, et de façon optionnelle un liant.

12. Méthode selon la revendication 11 consistant à mélanger 62 à 98 % de bio aragonite biocompatible avec 38 à 2 % de carbonate de calcium, par poids de composition à l'exception du liant.

## Patentansprüche

1. Biomaterial zur Behandlung von Parodontopathien und allen damit verbundenen Leiden, **dadurch gekennzeichnet, dass** es Folgendes umfasst:
- biokompatiblen Bio-Aragonit,
- reines Kalziumcarbonat, das in Kristallform oder in amorpher Form zugegeben wird,
- eventuell ein Bindemittel.

2. Biomaterial gemäß Anspruch 1, **dadurch gekennzeichnet, dass** das reine Kalziumcarbonat, das zugegeben wird, in pulverförmiger Form vorliegt.

3. Biomaterial gemäß Anspruch 1 oder 2, **dadurch gekennzeichnet, dass** der biokompatible Bio-Aragonit in einem Verhältnis von 62 bis 98 Gew.-%, vorzugsweise in einem Verhältnis von 70 bis 90 Gew.-% und am besten von 75 bis 85 % des Gesamtgewichts des Biomaterials vorliegt.

4. Biomaterial gemäß einem der Ansprüche 1 bis 3, **dadurch gekennzeichnet, dass** das reine Kalziumcarbonat, das zugegeben wird, in einem Verhältnis von 2 bis 38 Gew.-%, vorzugsweise in einem Verhältnis von 10 bis 30 Gew.-% und am besten von 15 bis 25 % des Gesamtgewichts des Biomaterials vorliegt.

5. Biomaterial gemäß einem der Ansprüche 1 bis 4, **dadurch gekennzeichnet, dass** das Bindemittel aus Hyaluronsäure, Schwefelchondroitinsäure, Guar-Gummi, einem Alginat, Xanthangummi oder Kollagen ausgewählt wird.

6. Biomaterial gemäß einem der Ansprüche 1 bis 5, **dadurch gekennzeichnet, dass** das Bindemittel in einem Verhältnis von 6 bis 15 Gew.-%, vorzugsweise in einem Verhältnis von 7 bis 12 Gew.-% und am besten von 8 bis 10 % des Gesamtgewichts des Biomaterials vorliegt.

7. Biomaterial gemäß einem der Ansprüche 1 bis 6, **dadurch gekennzeichnet, dass** sich der biokompatible Bio-Aragonit aus der innersten Schicht der Muschelschale in mikronisierter Form zusammensetzt und bei der der immunogene Teil der organischen Substanz deaktiviert worden ist.

8. Biomaterial gemäß einem der Ansprüche 1 bis 7, **dadurch gekennzeichnet, dass** der biokompatible Bio-Aragonit mittels einer schonenden Denaturierung der inneren Schicht der Muschelschale erhalten wird, wobei die Denaturierung derart ist, dass der immunogenste Teil der organischen Substanz dieser inneren Schicht deaktiviert wird und dass der Teil der organischen Substanz, der mit dem Mineral in Verbindung steht, das eine positive Wirkung aufweisen kann, im Wesentlichen erhalten bleibt, gefolgt von Spülen, Trocknen und Zerstoßen bis zum Erhalt einer pulverförmigen Form.

9. Biomaterial gemäß Anspruch 8, **dadurch gekennzeichnet, dass** die schonende Denaturierung aus einer Oxidation des immunogensten Teils der organischen Substanz durch Hypochlorit besteht.

10. Die Verwendung eines Gemisches aus biokompatiblem Bio-Aragonit und in Kristallform oder in amorpher Form zugegebenem Kalziumcarbonat zur Herstellung eines Biomaterials, das zur Behandlung von Parodontopathien und allen damit verbundenden Leiden bestimmt ist.

11. Ein Verfahren zur Vorbereitung einer Zusammensetzung, die einen biokompatibten Bio-Aragonit zur Behandlung von Parodontopathien und allen damit verbundenen Leiden und wahlweise ein Bindemittel enthält, wobei das Verfahren aus dem Mischen des biokompatiblen Bio-Aragonits mit reinem Kalziumcarbonat in Kristallform oder in amorpher Form besteht.

12. Verfahren gemäß Anspruch 11, das aus dem Mischen von 62 bis 98 % biokompatiblem Bio-Aragonit mit 38 bis 2 % Kalziumcarbonat pro Gewicht der Zusammensetzung ohne das Bindemittel besteht.

## Claims

1. A biomaterial for treatment of periodontal diseases and all related affections, **characterised in that** it includes:
- biocompatible bioaragonite,
- added pure calcium carbonate in crystalline or amorphous form,
- possibly a binder.

2. The biomaterial according to Claim 1, **characterised in that** the added pure calcium carbonate is in a powdery form.

3. The biomaterial according to one of Claims 1 or 2, **characterised in that** the biocompatible bioaragonite is present in a proportion of 62% to 98% by weight, preferentially in a proportion of 70% to 90% and yet more preferentially of 75% to 85% in relation to the biomaterial total weight.

4. The biomaterial according to one of Claims 1 to 3, **characterised in that** the added pure calcium carbonate is present in a proportion of 2% to 38% by weight, preferentially in a proportion of 10% to 30% and yet more preferentially of 15% to 25% in relation to the biomaterial total weight.

5. The biomaterial according to any one of Claims 1 to 4, **characterised in that** the binder is selected from hyaluronic acid, sulphuric chondroitin acid, guar gum, alginate, xanthan gum or collagen.

6. The biomaterial according to any one of Claims 1 to 5, **characterised in that** the binder is present in a proportion of 6% to 15% by weight, preferentially in a proportion of 7% to 12% and yet more preferentially of 8% to 10% in relation to the biomaterial total weight.

7. The biomaterial according to any one of Claims 1 to 6, **characterised in that** the biocompatible bioaragonite is made up by the innermost layer of a bivalve mollusc shell in micronised form, in which the immunogenic part of the organic substance has been inactivated.

8. The biomaterial according to any one of Claims 1 to 7, **characterised in that** the biocompatible bioaragonite is obtained by means of a controlled denaturation of the bivalve mollusc shell inner layer, the denaturation being such that the most immunogenic part of the organic substance of this inner layer is inactivated and that the part of the organic substance associated with the mineral likely to have a positive effect is essentially preserved, followed by rinsing, drying and crushing until obtaining a powdery form.

9. The biomaterial according to Claim 8, **characterised in that** the controlled denaturation consists of oxidation of the most immunogenic part of the organic substance with hypochlorite.

10. The use of a mixture of biocompatible bioaragonite and added pure calcium carbonate in crystalline or amorphous form to produce a biomaterial for the treatment of periodontal diseases and all related affections.

11. A method of preparing a composition containing biocompatible bioaragonite for the treatment of periodontal diseases and all related affections, which consists in mixing the biocompatible bioaragonite with pure calcium carbonate in crystalline or amorphous form and, optionally a binder.

12. The method according to Claim 11, consisting in mixing 62% to 98% of biocompatible bioaragonite with 38% to 2% of calcium carbonate, by weight of the composition excluding the binder.
